# EUROPEAN PATENT SPECIFICATION

(11) **EP 0 456 793 B1**
(45) Date of publication and mention of the grant of the patent: **23.10.1996**
(21) Application number: 90917731.3
(22) Date of filing: 03.12.1990
(51) Int. Cl.: C07D 213/26, C09K 19/34, C07D 239/26, C07D 239/34

(54) **HALOPHENYL SUBSTITUTED HETEROCYCLIC DERIVATIVES**
HALOPHENYL-SUBSTITUIERTE HETEROZYKLISCHE DERIVATE
DERIVES HETEROCYCLIQUES SUBSTITUES PAR HALOPHENYLE

(30) Priority: 08.12.1989 GB 8927779; 08.12.1989 GB 8927780; 01.02.1990 DE 4002860
(43) Date of publication of application: 21.11.1991
(73) Proprietor: MERCK PATENT GmbH, D-64271 Darmstadt (DE)
(72) Inventor: COATES, David 87 Sopwith Crescent Merley, Dorset BH 21 3SW (GB); SMITH, Graham 10 Henbury Close Canford Heath, Dorset BH17 8AX (GB); GREENFIELD, Simon 2 Blackbird Close, Poole BH17 7YA (GB); KRAUSE, Joachim, D-6110 Dieburg (DE); HITTICH, Reinhard, D-6101 Modautal (DE); POETSCH, Eike, D-6109 Mühltal 6 (DE); RIEGER, Bernhard, D-6115 Münster (DE)
(86) International application number: EP9002071
(87) International publication number: WO9109016

(56) References cited:
- EP-A- 0 123 907
- EP-A- 0 194 153
- EP-A- 0 240 320
- WO-A-87/04158
- GB-A- 1 247 361
- JP-A- 6 210 083
- Chemical Abstracts, vol. 110, no. 24, 12 June 1989, (Columbus, Ohio, US), see page 722

## Description

The invention relates to a liquid crystalline medium being a mixture of at least two compounds at least one compound is a chlorophenyl substituted heterocyclic compound of formula I 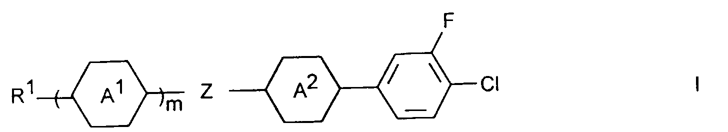 in which
- R¹: is an alkyl or alkenyl residue each with up to 16 C atoms, wherein one or two non-adjacent CH₂ groups may be replaced by -O-, -S-, -CO-O-
or
-O-CO-,
- A²: is a heterocyclic ring of formula 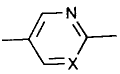 wherein X is N or CH,
- A¹: is 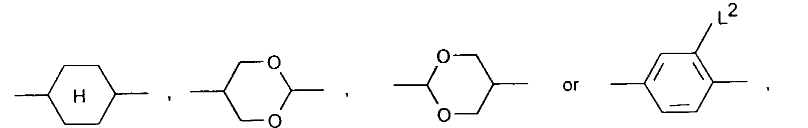
- L²: is H or F,
- Z: is (CH₂)ₙ or -COO-,
- m: is 0 or 1, and
- n: are 0 or 2.

The compounds of the formula I can be used as components of liquid crystal media, in particular for displays which are based on the principle of the twisted nematic cell, including

TN cells with a higher twist angle like SIN, SBE, OMI., etc., on the guest-host effect, on the effect of deformation of orientated phases or on the effect of dynamic scattering.

Compounds similar to those of formula I are known from EP 0 123 907. These compounds comprise 2 rings but in any case a non-fluorinated phenylene group and lead to liquid crystal media with a unfavorable high threshold due to their low values of the dielectric anisotropy.

Compounds of the formula I with three rings and L = H or F are claimed in EP 0 152 808, EP 0 158 137, EP 0 199 004, EP 0 256 636 via very broad formulae. There are, however, no specific examples for these compounds so that it may be stated that the surprisingly advantageous properties of these compounds have neither been realized nor used.

Similar halophenylpyridine derivatives without lateral fluorine substitution are known: 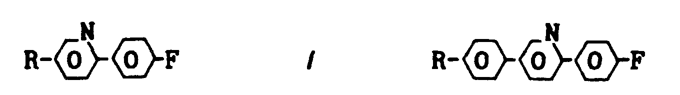 JP 62/096 469-A 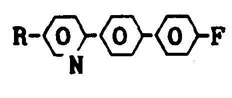 JP 63 063 662-A

The following laterally fluorinated derivatives exhibiting no ethyl-linkage are also known 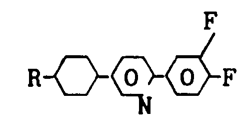 US 4,772,416 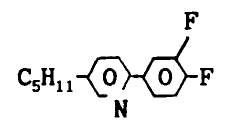 EP 01 94 159

Furthermore the compounds of the formula I are embraced by the broad speculative formula of the International Patent WO 87104158 but there is no hint in this application to the terminally halogenated compounds of the formula I.

Similar compounds of the formula 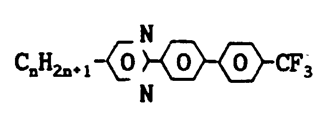 are known from EP-0 193 191 but these exhibit melting points higher than 125 °C and have only smectic phases.

The invention was based on the object of discovering new stable liquid crystalline or mesogenic compounds which are especially suitable for active matrix displays without exhibiting the shortcomings of the prior art compounds.

Surprisingly, it has been found that the compounds according to formula I meet these criteria to an outstanding degree and allow the realization of a very high resistivity and simultaneously favorable electro-optic characteristics. These compounds also show a surprisingly good solubility at -20 °C in many LC base materials from other compound classes.

By providing the compounds of the formula I, the range of liquid crystal substances which are suitable, under various technological aspects for the preparation of nematic mixtures is also quite generally widened considerably.

The compounds of the formula I have a wide field of application and they are preferably added to liquid crystal base materials from other classes of compounds, for example in order to increase the clearing point while having little effect on other physical properties.

The compounds of the formula I are in particular suitable as components of liquid crystal media for displays which are based on the principle of polymer dispersed liquid crystals (pdlc) or polymer network liquid crystals (pnlc) due to their favorable optical and dielectric anisotropy.

The compounds of the formula I can be used like similar known compounds (e.g. DE 27 02 591) as components of liquid crystalline media, in particular for displays which are based on the principle of the twisted nematic cell.

The compounds used for this application until now have some disadvantages as, for example, high melting points, low clearing points, low stability against heat, light or electric fields, low electric resistivity, high temperature dependences of the threshold voltage.

In particular in displays of the supertwist-type (STN) with tilt angles more than 220 degree or displays with active matrix the materials known until now have many drawbacks.

The problem of this invention was to find new liquid crystalline compounds suitable as components of liquid crystalline media, in particular nematic media with a positive dielectric anisotropy which do not show the disadvantages of the known ones. The solution of this problem are the new compounds of the formula I.

It has been found that the compounds of the formula I are suitable as components of liquid crystalline media.

In particular, they allow to prepare liquid crystalline media, with a broad nematic phase range, excellent nematogenity also at low temperatures, excellent chemical stability, high ε┴ and positive dielectric anisotropy, accordingly relative low Δε/ε┴, low temperature dependance of the threshold voltage and/or high optic anisotropy. The new compounds show, additionally a good solubility for other components of liquid-crystalline media and a high positive dielectric anisotropy combined with favorable viscosity.

The compounds of the formula I are colourless in the pure state and form liquid crystal mesophases in a temperature range which is favorably placed for electrooptical use. They are very stable towards chemicals, heat and light.

The invention relates to liquid crystal media containing at least one compound of the formula I and to liquid crystal display elements, in particular electrooptical display elements, which contain media of this type.

Above and below R¹-, 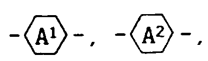 L², m and n have the mentioned meaning, unless expressly stated otherwise.

Furthermore the pyrimidine derivatives of the formula Ia and Ib are preferred: 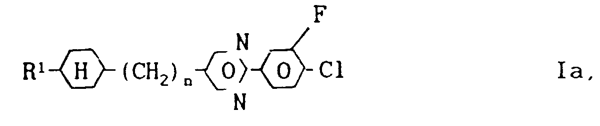 Hal is F,

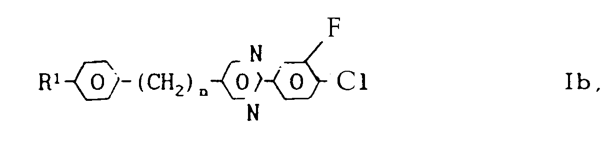

The compounds of the formula If1 and If4 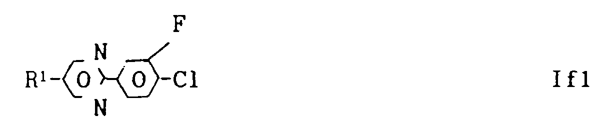 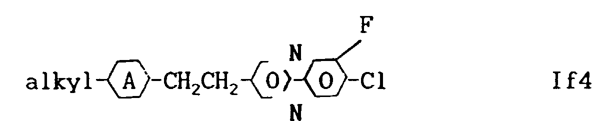 are preferred.

The preferred compounds of the formula Ig 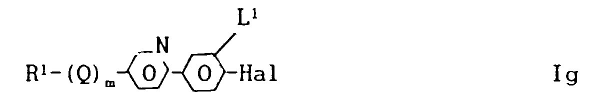 wherein Q and m have the meaning given include pyridine derivatives of the formulae Ig1 to Ig2 : 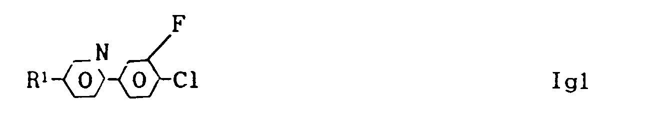 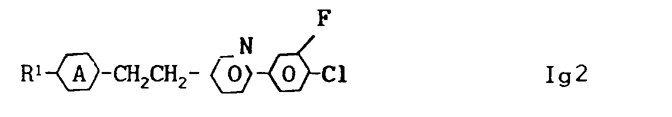 R¹ is preferably alkyl, alkoxy, oxaalkyl or alkenyl and can exhibit a straight-chain or branched structure.

Alkyl or alkoxy preferably are straight-chain and have 2, 3, 4, 5, 6 or 7 C atoms. Accordingly they are preferably ethyl, propyl, butyl, pentyl, hexyl, heptyl, ethoxy, propoxy, butoxy, pentoxy, hexoxy or heptoxy, also methyl, octyl, nonyl, decyl, undecyl, dodecyl, tridecyl, tetradecyl, pentadecyl, methoxy, octoxy, nonoxy, decoxy, undecoxy, dodecoxy, tridecoxy or tetradecoxy.

Oxaalkyl is preferably straight-chain 2-oxapropyl (= methoxymethyl), 2-( = ethoxymethyl) or 3-oxybutyl (= 2-methoxyethyl), 2-, 3- or 4-oxapentyl, 2-, 3-, 4- or 5-oxahexyl, 2-, 3-, 4-, 5- or 6-oxaheptyl, 2-, 3-, 4-, 5-, 6- or 7-oxaoctyl, 2-, 3-, 4-, 5-, 6-, 7- or 8-oxanonyl, 2-, 3-, 4-, 5-, 6-, 7-, 8- or 9-oxadecyl.

Alkenyl is preferably straight-chain and has 2 to 10 C atoms. It is accordingly, in particular, vinyl, prop-1- or prop-2-enyl, but-1-, -2- or -3-enyl, pent-1-, -2-, -3- or -4-enyl, hex-1-, -2-, -3-, -4- or -5-enyl, hept-1-, -2-, -3-, -4-, -5-or -6-enyl, oct-1-, -2-, -3-, -4-, -5-, -6- or -7-enyl, non-1-, -2-, -3-, -4-, -5-, -6-, -7- or -8-enyl, dec-1-, -2-, -3-, -4-, -5-, -6-, -7-, -8- or -9-enyl.

Compounds of the formula I containing a branched terminal group can occassionally be of importance because of an improved solubility in the customary liquid crystal base materials, but in particular as chiral doping substances if they are optically active.

In the compounds of these formulae R¹ is preferably alkyl, furthermore also groups in which one CH₂-group is replaced by oxygen.

If R¹ is alkyl and/or alkoxy groups, these can be straight-chain or branched. Preferably, they are straight-chain and have 2, 3, 4, 5, 6 or 7 C atoms and are accordingly preferably ethyl, propyl, butyl, pentyl, hexyl, heptyl, ethoxy, propoxy, butoxy, pentoxy, hexoxy or heptoxy, also methyl, octyl, nonyl, methoxy, octoxy or nonoxy.

Oxaalkyl is preferably straight-chain 2-oxapropyl (= methoxymethyl), 2- (= ethoxymethyl) or 3-oxybutyl (= 2-methoxyethyl), 2-, 3- or 4-oxapentyl, 2-, 3-, 4- or 5-oxahexyl, 2-, 3-, 4-, 5- or 6-oxaheptyl, or 2-, 3-, 4-, 5-, 6- or 7-oxaoctyl.

Compounds of the formula I with branched terminal group R¹ can be of importance because of a better solubility in the customary liquid crystal base materials, but in particular as chiral doping substances if they are optically active.

Branched groups of this type as a rule contain not more than one chain branching. Preferred branched groups are isopropyl, 2-butyl (= 1-methylpropyl), isobutyl (= 2-methylpropyl), 2-methylbutyl, isopentyl (= 3-methylbutyl), 2-methylpentyl, 3-methylpentyl, 2-ethylhexyl, 2-propylpentyl, 2-octyl, isopropoxy, 2-methylpropoxy, 2-methylbutoxy, 3-methylbutoxy, 2-methylpentoxy, 3-methylpentoxy, 2-ethylhexoxy, 1-methylhexoxy, 1-methylheptoxy (= 2-octyloxy), 2-oxa-3-methylbutyl, 3-oxa-4-methylpentyl, 4-methylhexyl and 6-methyloctoxy.

In the case of compounds with branched terminal group, formula I includes both the optical antipodes and racemates as well as mixtures thereof.

Of the compounds of the formula I and subformulae thereof, those in which at least one of the radicals contained therein has one of the preferred meanings given are preferred.

The group 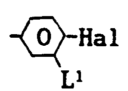 denotes

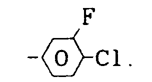

The compounds of the formula I are prepared by methods which are known per se, such as are described in the literature (for example in the standard works, such as Houben-Weyl, Methoden der Organischen Chemie [Methods of Organic Chemistry], Georg-Thieme-Verlag, Stuttgart), and in particular under reaction conditions which are known and suitable for the reactions mentioned. Variants which are known per se and are not mentioned in more detail here can also be used in this connection.

If desired, the starting substances can also be formed in situ, such that they are not isolated from the reaction mixture but are immediately reacted further to give the compounds of the formula I.

The preferred synthetic routes are shown in the following schemes (I to VIII):

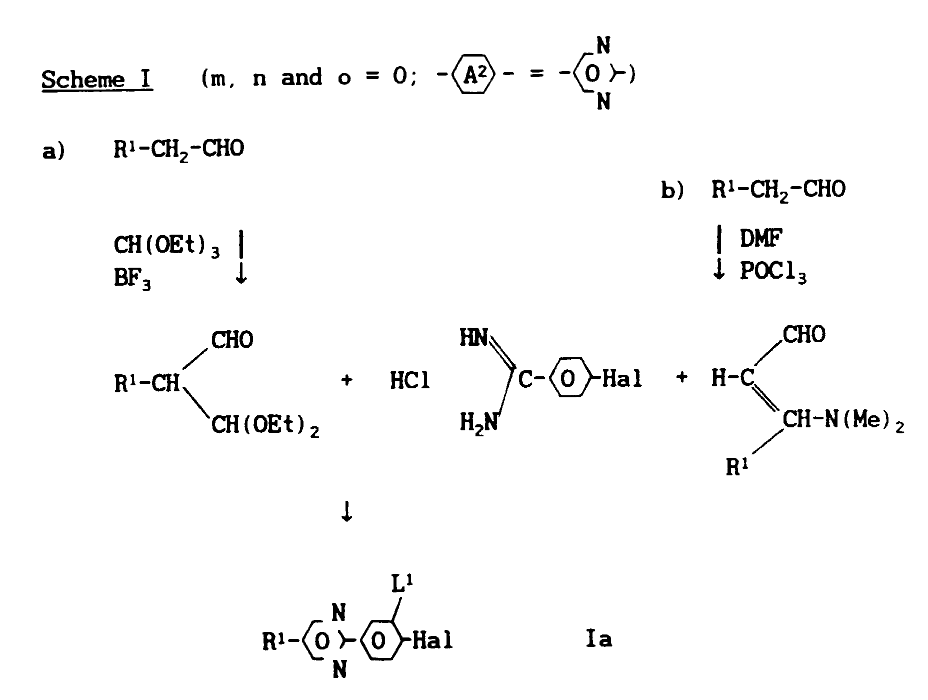

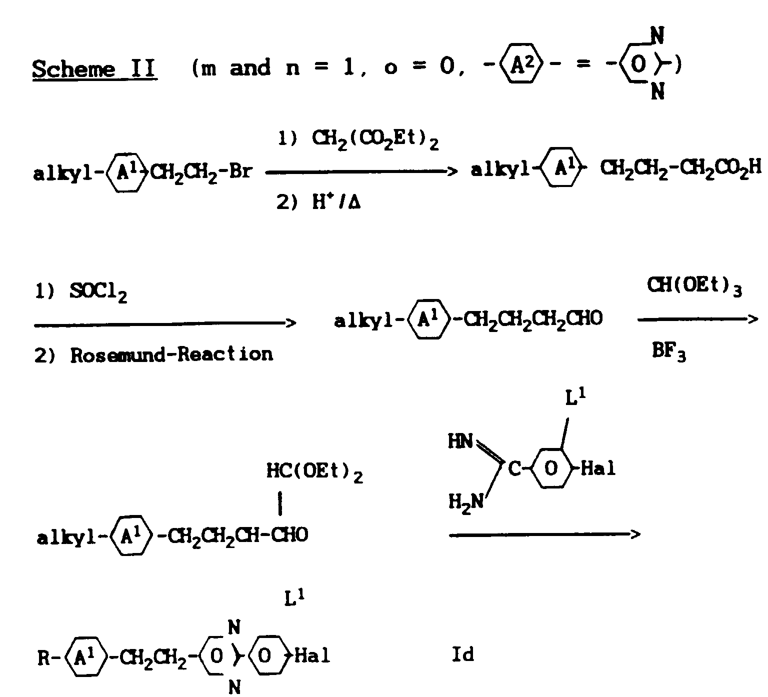

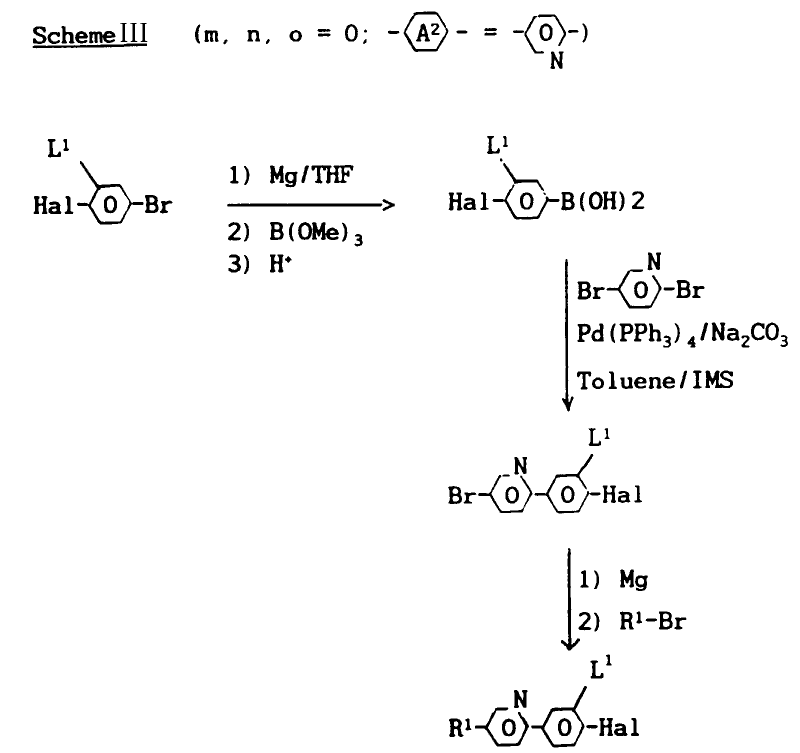

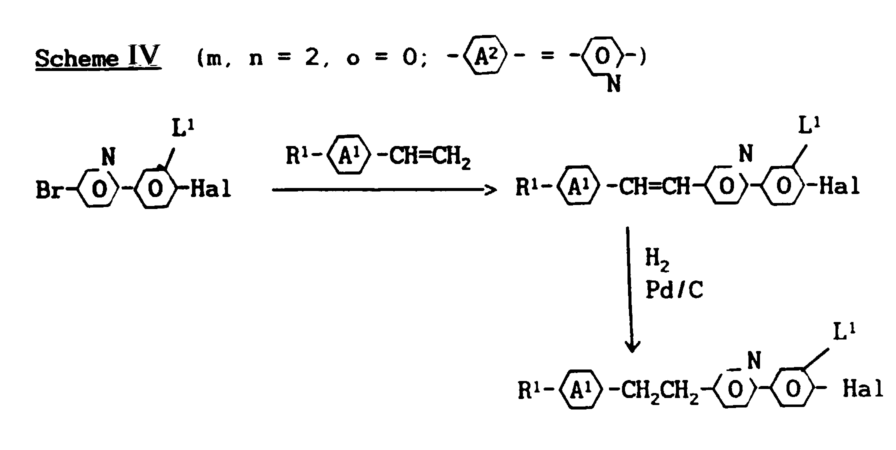

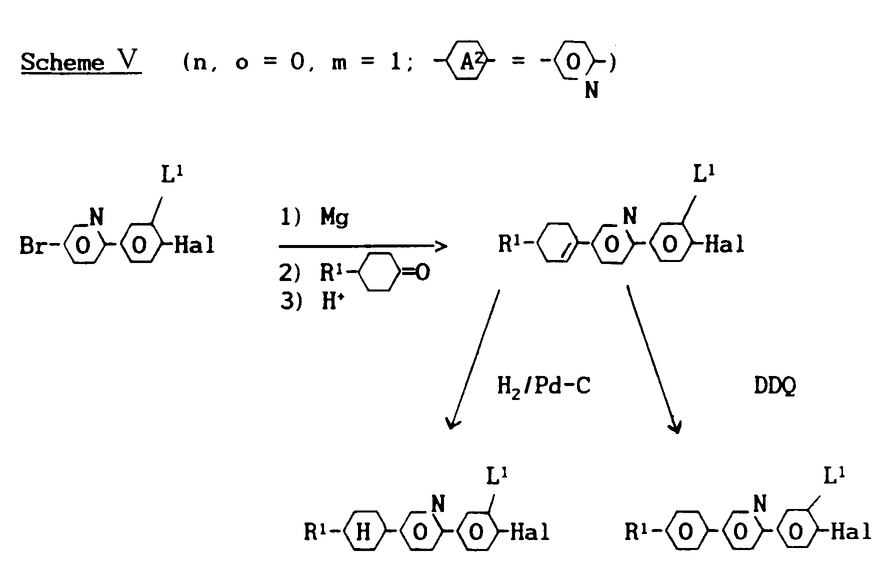

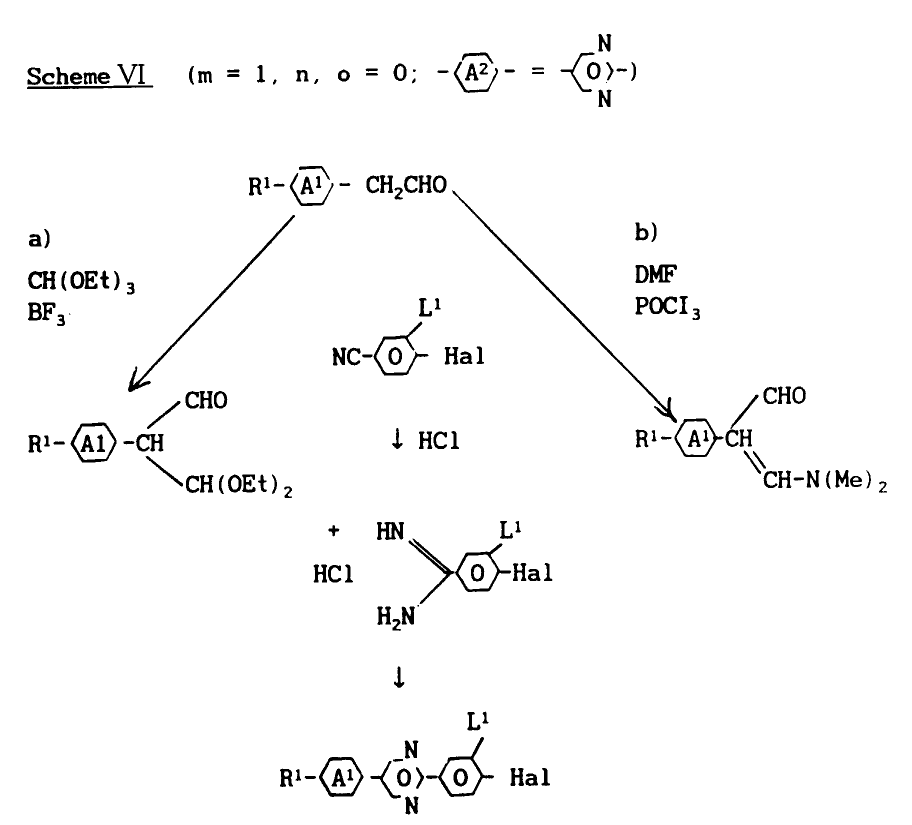

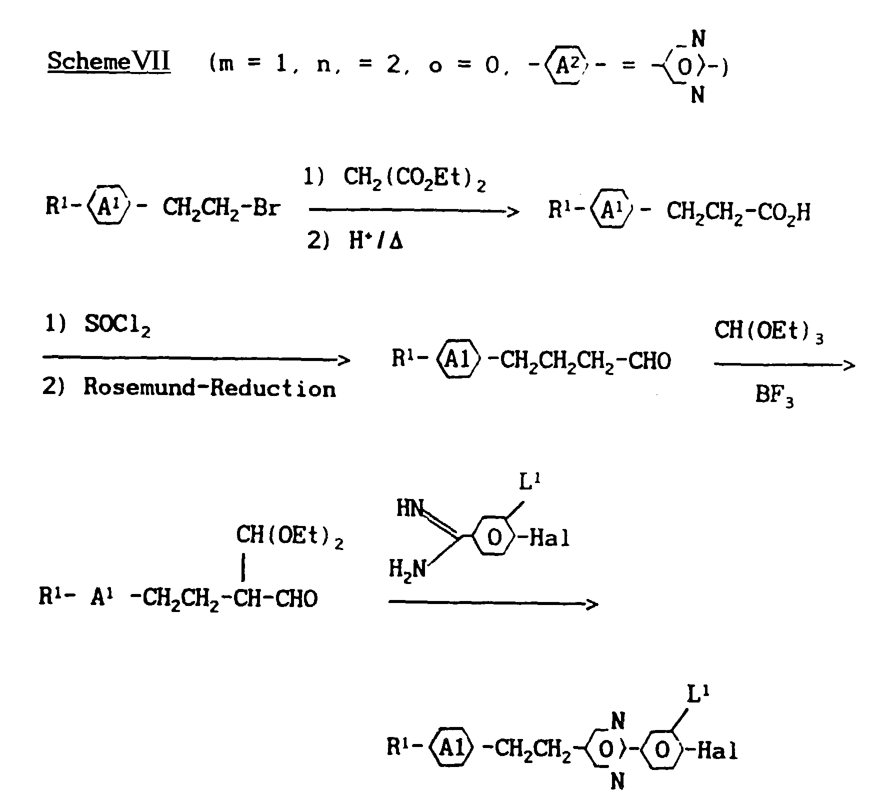

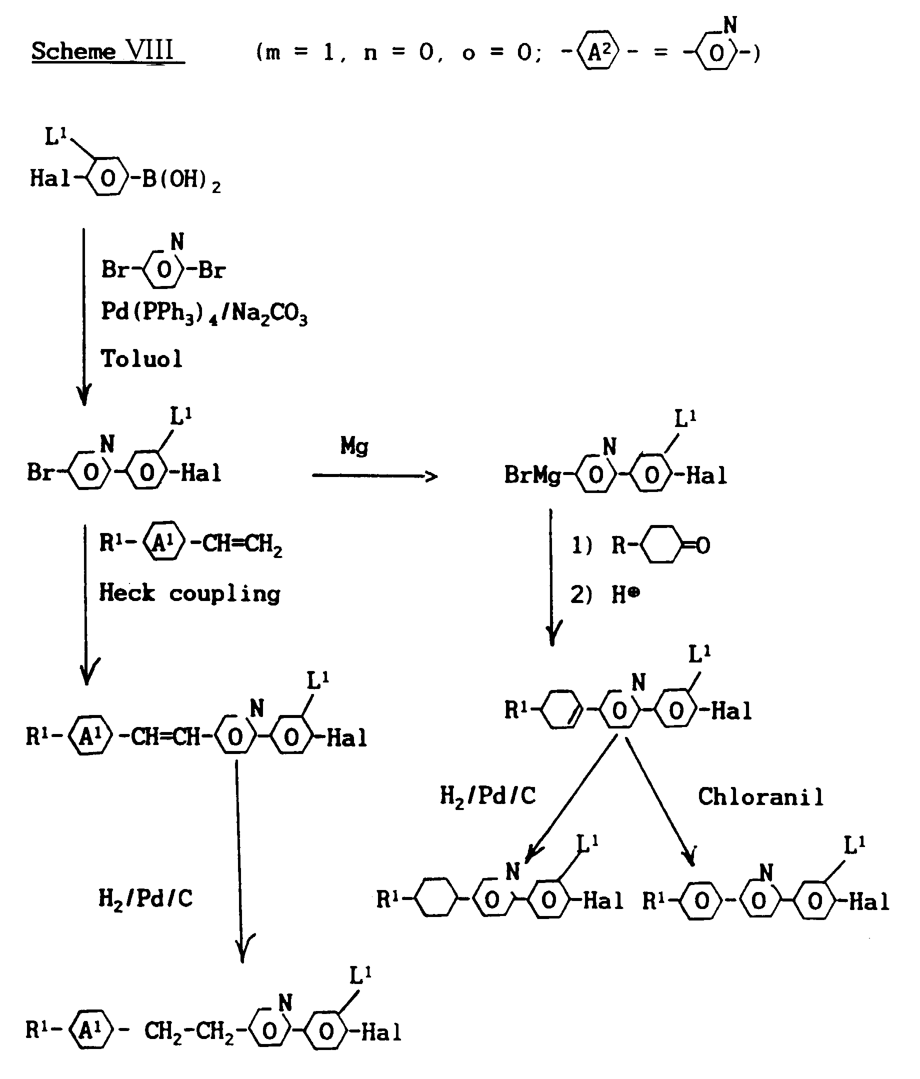

These additional components are preferably chosen from the nematic or nematogenic (monotropic or isotropic) substances; in particular from the classes of azoxybenzenes, benzylideneanilines, biphenyls, terphenyls, phenyl or cyclohexyl benzoates, phenyl or cyclohexyl cyclohexanecarboxylates, phenyl or cyclohexyl cyclohexylbenzoates, phenyl or cyclohexyl cyclohexylcyclohexanecarboxylates, cyclohexylphenylbenzoates, cyclohexylphenyl cyclohexanecarboxylates, cyclohexylphenyl cyclohexylcyclohexanecarboxylates, phenylcyclohexanes, cyclohexylbiphenyls, phenylcyclohexylcyclohexanes, cyclohexylcyclohexanes, cyclohexylcyclohexenes, cyclohexylcyclohexylcyclohexene, 1,4-bis-cyclohexylbenzenes, 4,4'-bis-cyclohexylbiphenyls, phenyl- or cyclohexylpyrimidines, phenyl- or cyclohexylpyridines, phenyl- or cyclohexyldioxanes, phenyl-or cyclohexyl-1 3-dithianes, 1,2-diphenylethanes, 1,2-dicyclohexylethanes, l-phenyl-2-cyclohexylethanes, 1-cyclohexyl-2-(4-phenyl-cyclohexyl)-ethanes, 1-cyclohexyl-2-biphenylethanes, 1-phenyl-2-cyclohexyl-phenylethanes, optionally halogenated stilbenes, benzyl phenyl ethers, tolanes and substituted cinnamic acids.

The 1,4-phenylene groups of these compounds may be fluorinated.

The most important compounds which are possible constituents of liquid crystal media according to the invention can be characterized by the formalae 1, 2, 3, 4 and 5:

R'-L-E-R" 1

R'-L-COO-E-R" 2

R'-L-OOC-E-R" 3

R'-L-CH₂CH₂-E-R" 4

R'-L-C=C-E-R" 5

In the formulae 1, 2, 3, 4 and 5 L and E may be equal or different from each other. L and E independently from each other denote a bivalent residue selected from the group consisting of -Phe-, -Cyc-, -Phe-Phe-, -Phe-Cyc-, -Cyc-Cyc-, -Pyr-, -Dio-, -G-Phe-, -G-Cyc- and their mirror images; in this compilation of residues Phe denotes unsubstituted or fluorinated 1,4-phenylen, Cyc trans- 1,4-cyclohexylene or 1,4-cyclohexenylen, Pyr pyrimidine-2,5-diyl or pyridine-2,5-diyl, Dio 1,3-dioxane-2,5-diyl and G 2-(trans-1,4-cyclohexyl)-ethyl, pyrimidine-2,5-diyl, pyridine-2,5-diyl or 1,3-di-oxane-2,5-diyl.

One of the residues L and E is preferably Cyc, Phe or Pyr. E preferably denotes Cyc, Phe or Phe-Cyc. The liquid crystal media according to the invention preferably contain one or more components selected from the compounds of formulae 1, 2, 3, 4 and 5 with L and E meaning Cyc, Phe and Pyr, said liquid crystal media further containing at the same time one or more components selected from the compounds of formulae 1, 2, 3, 4 and 5 with one of the residues L and E denoting Cyc, Phe and Pyr and the other residue being selected from the group consisting of -Phe-Phe-, -Phe-Cyc-, -Cyc-Cyc-, -G-Cyc-, said liquid crystal media containing in addition to this optionally one or more components selected from the compounds of formulae 1, 2, 3, 4 and 5 with L and E being selected from the group consisting of -Phe-Cyc-, -Cyc-Cyc-, -G-Phe- and -G-Cyc.

In a preferred subgroup of the compounds of formulae 1, 2, 3, 4 and 5 (subgroup 1) R' and R" are independently from each other alkyl, alkenyl, alkoxy, alkenoxy with up to 8 carbon atoms. R' and R" differ from one another in most of these compounds, one of the residues usually being alkyl or alkenyl. In another preferred subgroup of the compounds of formulae 1, 2, 3, 4 and 5 (subgroup 2) R" denotes -CN, -CF₃, -OCF₃-,-OCHF₂, -F, -Cl or -NCS while R' has the meaning indicated in subgroup 1 and is preferably alkyl or alkenyl. Other variants of the envisaged substituents in the compounds of formulae 1, 2, 3, 4 and 5 are also customary. Many such substances are commercially available. All these substances are obtainable by methods which are known from the literature or by analogous methods.

The liquid crystal media according to the invention preferably contain in addition to components selected from subgroup 1 also components of subgroup 2, the percentage of these components being as follows:
- subgroup 1:: 20 to 90 %, in particular 30 to 90 X
- subgroup 2:: 10 to 50 %, in particular 10 to 50 %

In these liquid crystal media the percentages of the compounds according to the invention and the compounds of subgroup 1 and 2 may add up to give 100 %.

The media according to the invention preferably contain 1 to 30 %, in particular 5 to 20 % of the compounds according to the invention. The media contain preferably 3, 4 or 5 compounds according to the invention.

The media according to the invention are prepared in a manner which is customary per se. As a rule, the components are dissolved in one another, advantageously at elevated temperature. The liquid crystal media according to the invention can be modified by suitable additives so that they can be used in all the types of liquid crystal display devices. Such additives are known to the expert and are described in detail in the literature (H. Kelker/R. Hatz, Handbook of Liquid Crystals, Verlag Chemie, Weinheim, 1980). For example, it is possible to add pleochroic dyestuffs to prepare colored guest-host systems or substances for modifying the dielectric anisotropy, the viscosity and for the orientation of the nematic phases.

The following examples are to be construed as merely illustrative and not limitative. m.p. = melting point, c.p. = clearing point. In the foregoing and in the following all parts and percentages are by weight and the temperatures are set forth in degrees Celsius. "Customary work-up" means that water is added, the mixture is extracted with methylene chloride, the organic phase is seperated off, dried and evaporated, and the product is purified by crystallization and for chromatography.

Further are:
C: crystalline-solid state, S: smectic phase (the index denoting the typ of smectic phase), N: nematic phase, Ch: cholesteric phase, I: isotropic phase. The number being embraced by 2 of these symbols denotes the temperature of phase change.

### Example 1

### Preparation of 5-propyl-2-(4-chloro-3-fluorophenyl)-pyrimidine

4-Chloro-3-fluorobenzamidine hydrochloride (0.05 mol obtained from 4-chloro-3-fluorobenzonitrile) is added to 3,3-di-ethoxy-2-propionylaldehyde (0.1 mole) and dimethylformamide (50 ml) The reaction mixture is heated to 130 °C for 4 hours and a distillate collected. DMF (20 ml) was added and the mixture heated for 16 hrs at 130 °C. Solvent is distilled off and the crude mixture taken up in ether and washed with water. The pure propduct is obtained by repeated crystallization from methanol, C 71 I, Δε 40.2.

### Example 2 to 3

Example 1 is repeated except that 2-propyl-3-dimethylamineacrolein of Example 1 is replaced by 2-alkyl-3-dimethylaminoacroleines to prepare compounds shown in Example 2 to 6 of Table I

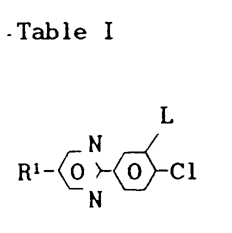

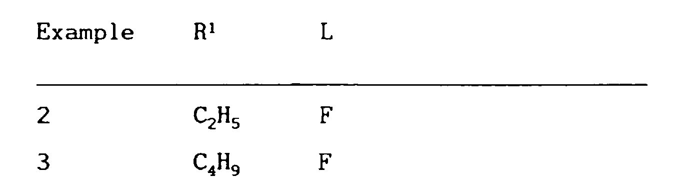

### Example 4

### Preparation of 5-Pentyl-2-(4-chloro-3-fluorophenyl)-pyridine

### Step 1

### 4-Chloro-3-fluorophenylboronic acid

Magnesium (6.4 g, 0.263 moles) and THE (150 ml) are stirred under nitrogen. 1-bromo-3-fluoro-4-chlorobenzene (50 g, 0.239 moles) is added dropwise to the reaction mixture which is left to stir and reflux for 2 hrs. The reaction mixture is cooled to 0 °C, and trimethyl borate (30 ml, 0.263 moles) in THF (50 ml) is added keeping the temperature below 20 °C. The reaction mixture is left to stir in the ice bath for 30 mins, then quenched with acid solution (45 ml cHCl, 80 ml H₂O). The reaction mixture is extracted into ether, washed well with H₂O, then brine and finally the ether was evaporated to afford the crude boronic acid.

### Step 2

### 5-Bromo-2-(4-chloro-3-fluorophenyl)-pyridine

A solution of the boronic acid of STEP 1 (0.01 mol) in ethanol (20 ml) is added dropwise to a stirred mixture of 2,5-dibromopyridine (0,01 mol), tetrakis(triphenylphosphine)-palladium(0) (0.01 mmol) in toluene (25 ml) and 2 M-Na₂CO₃ (25 ml) at room temperature under dry N₂. The stirred mixture is heated under reflux for 24 h. The mixture is cooled, then stirred with 30 % H₂O₂ (2 ml) for 2 h at room temperature. The product is extracted into ether twice and the combined organic extracts are washed with brine and dried. The solvent is removed and the residue is purified by column chromatography.

### Step 3

A solution of n-pentylbromide (0.01 mol) in dry THF (10 ml) is added to a stirred, cooled solution of the Grignard Reagent prepared from the product of STEP 2 (0.008 mol) and Mg (0.01 mol) in dry THF (30 ml) under dry N₂. The stirred mixture is allowed to warm up to room temperature and stirred with 10 % HCl (200 ml) The product is extracted into ether twice, and the combined etheral extracts are washed with water and dried. Solvent is removed in vacuo to yield the desired product.

### Examples 5 to 9

In place of the used pentylbromide and the 4-chloro-3-fluorobromobenzene other starting materials can be used for the preparation of the following compounds in analogy to example 4:

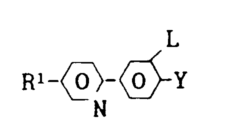

| | | | | |
|---|---|---|---|---|
| Example | R¹ | L | Y | |
| 5 | C₃H₇ | F | Cl | |
| 6 | CH₃ | F | Cl | C 58 I Δε = 11.6 |
| 7 | C₆H₁₃ | F | Cl | |
| 8 | C₇H₁₅ | F | Cl | |
| 9 | CH₂=CH-CH₂ | F | Cl | |

### Reference - Example 10

### Preparation of 5-[2-(trans-4-pentylcyclohexyl)-ethyl]-2-(4-chloro-2-fluorophenyl)-pyridine

With the product of Example 4 STEP 2 (0.1 mol) and trans-4-pentyl-1-vinylcyclohexane as starting material the desired product is obtained by Heck-coupling and hydrogenation according Scheme VI.

### Example 11 to 13

In place of the 4-chloro-3-fluoro-bromo-bencene and trans-4-pentyl-1-vinylcyclohexane other starting materials can be used for the preparation of the following compounds in analogy to reference example 10:

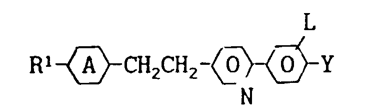

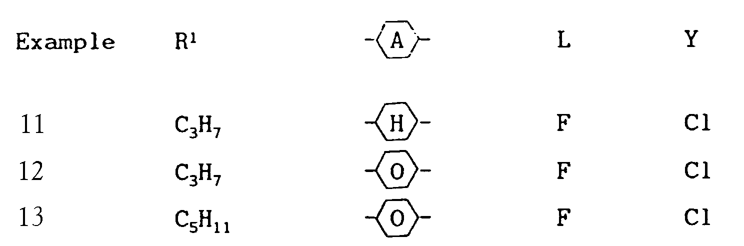

### Example 14

### 2-(4-Chloro-3-fluorophenyl)-5-(4-propylphenyl)-pyridine

A solution of 4-chloro-3-fluorophenylboronic acid (0.01 mol obtained from 4-chloro-3-fluorobromobenzene with magnesium and trimethylborate) in ethanol (20 ml) is added to a stirred mixture of 2,5-dibrompyridine (0.01 mol), tetrakis-(triphenylphosphin)palladium (0) (0.01 mol) in toluene (25 ml) at room temperature.

After costumary work-up 5-bromo-2-(4-chloro-3-fluorophenyl)-pyridine is made with 0.01 moles of magnesium to a Grignardreagences (according scheme XIII) (in THF/40 ml).

A mixture of 4-propylcyclohexanone (0.01 mol) and 20 ml THF is added to the solution of the Grignard reagence. After costumary work-up the crude product is diluted in ethanol (50 ml) and conc. H₂SO₄ and heated. The crude 2-(4-chloro-3-fluorophenyl)-5-(4-propylcyclohex-1-enyl)-pyridine is heated with chloramide (0.03 mol) in toluene. The pure product is obtained by repeated crystallization, C 77 S_{B} 95 S_{A} 183 I.

### Examples 15 to 16

Using other 5-bromo-(4-halogeno-3-Y-phenyl)-pyridines and other 4-alkylcyclohexanones the compounds of examples 15 to 16 are obtained (cp. Table II).

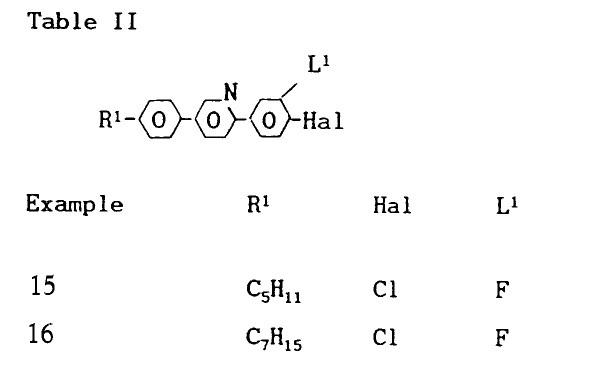

### Reference - Example 17

### 1-[2-(4-Fluorophenyl)-pyrimidine-5-yl]-2-(4-pentylphenyl)-ethane

4-Fluorobenzamidine hydrochlorid (0.05 mol, obtained from 4-fluorobenzonitrile) is added to 4-(4-pentylphenyl)-2-diethoxymethyl)-butyraldehyde (0.05 mol, obtained from 2-(4-pentylphenyl)-bromoethane and diethylmalonate according scheme X) and dimethylformamide to 50 ml. The mixture is heated for 4 h to 130 °C and after adding DMF 16 h to 130 °C. After customary work-up and repeated crystallization the pure product is obtained, C 100 I, Δε = 14.2.

### Examples 18 to 71

Reference - Example 17 is repeated using instead of benzamidine hydrochloride 4-halogeno-3-Y-benzamidines and instead of 4-(4-pentylphenyl)-2-(diethoxymethyl)-butyraldehyde 4-(4-alkylphenyl)- or 4-(trans-4-alkylcyclohexyl)-2-(diethoxymethyl)-butyraldehydes. The compounds of the Examples 18 to 21 are obtained (cp. Table III).

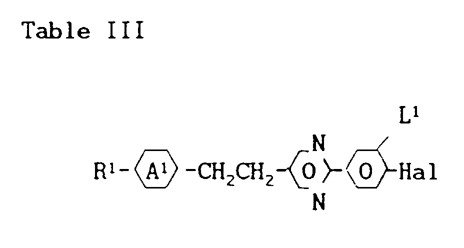

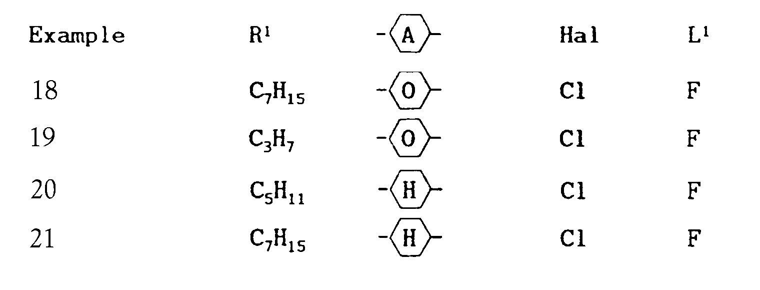

### Example 22

### 2-(4-Chloro-3-fluorophenyl)-5-(trans-4-propylcyclohexYl)-pyridine

A mixture of 0.01 mol 2-(4-Chloro-3-fluorophenyl)-5-(propylcyclohex-2-enyl)-pyridine (obtained as described in example 14), 50 ml toluene and 0.1 g Pd/C (10 %) is hydrogenated until saturation.

After filtration, destilling off of the solvent in vacuo and crystallization (twice) the pure product is obtained, C 142 N 191 I, Δε = 5.6.

### Example 23 to 24

Using other 2-(4-Chloro-3-Y-phenyl) -5-(alkylcyclohex-1-enyl)-pyridines the products of the examples 23 to 24 (cp. Table V) are obtained.

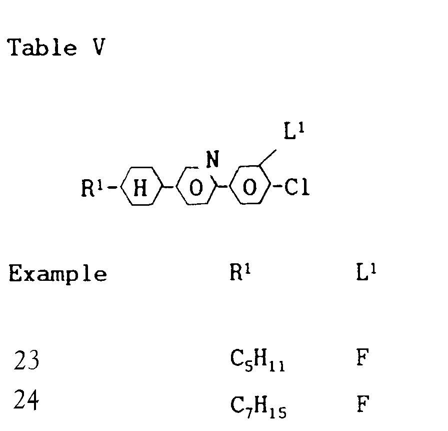

### Example 25

### 1-[2-(4-Chloro-3-fluorophenyl)-pyridine-5-yl]-2-(trans-4-pentylcyclohexyl)-ethane

A mixture of 0.01 mol 5-bromo-2-(4-chloro-3-fluorophenyl)-pyridine (obtained as described in example 42), 0.012 mol magnesium and 50 ml THF is heated under reflux.

A mixture of 0.011 mol 1-(trans-4-pentylcyclohexyl)-2-bromoethane and 20 ml THF is added to the solution. After stirring for 2 h at room temperature one works-up as usual. The pure product is obtained after repeated crystallization.

### Examples 26 to 94

Analogously the compounds of the examples 26 to 27 (cp. Table VI) are obtained.

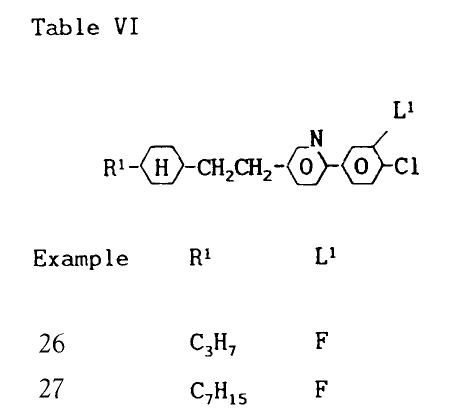

### Example 28

A solution of 2-chloro-5-[2-(trans-4-pentylcyclohexyl)-ethyl]-pyrimidine (0.01 mol obtained by palladium-catalyzed cross-coupling of 1-zincbromo-2- (trans-4-pentylcyclohexyl)-ethane with 2-(chloro-5-bromopyridine) in 20 ml toluene is added to a solution of 3-fluoro-4-chlorophenyl boronic acid (0.01 mol obtained by lithiation of 3-fluoro-4-chloro-bromobenzene followed by treatment with trimethylborate) and sodium carbonate (0.22 mol) in 10 ml water and 10 ml ethanol. 1,1'-Bis[diphenylphosphino]-ferrocen-palladium-II-chloride is added to the reaction mixture which is refluxed under stirring for 18 h.

After customary work-up and crystallization from ethanol 2-(3-fluoro-4-chlorophenyl)-5-[2-(trans-4-pentylcyclohexyl)-ethyl]-pyrimidine is obtained as colourless solid.

### Examples 29 to 103

The products of the examples 29 to 30 are obtained analogously (cp. Table VII).

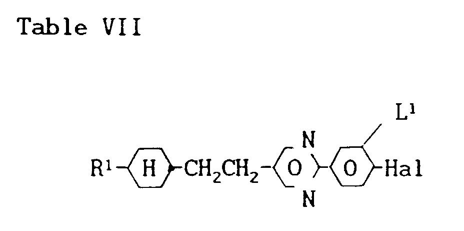

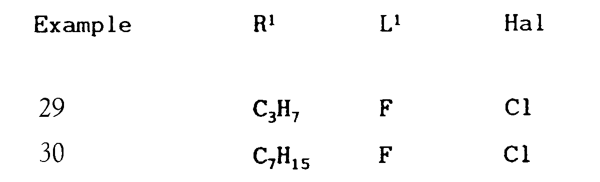

## Claims

1. Liquid crystalline medium being a mixture of at least two compounds at least one compound is a chlorophenyl substituted heterocyclic compound of formula I 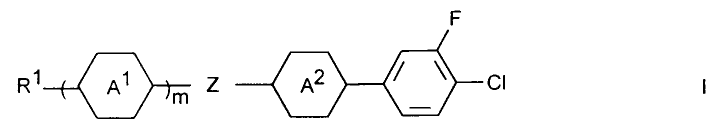 in which
R¹ is an alkyl or alkenyl residue each with up to 16 C atoms, wherein one or two non-adjacent CH₂ groups may be replaced by -O-, -S-, -CO-O-
or
-O-CO-,
A² is a heterocyclic ring of formula 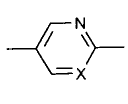 wherein X is N or CH,
A¹ is 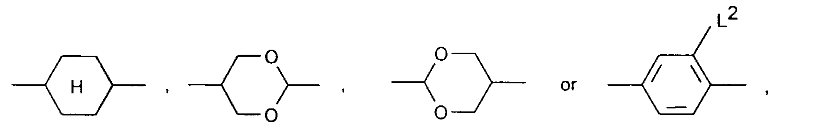
L² is H or F,
Z is (CH₂)ₙ or -COO-,
m is 0 or 1, and
n are 0 or 2.

2. Liquid crystalline medium according to claim 1 characterized in that the heterocyclic compound is selected from formulae Ia to Ib 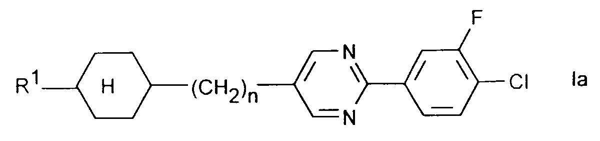 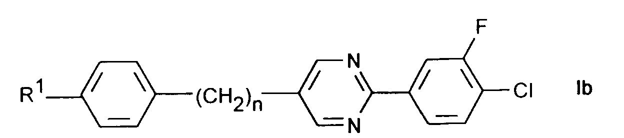 wherein R¹, and n have the meaning given.

3. Liquid crystalline medium according to claim 1 characterized in that the heterocyclic compound is selected from formulae If1 and If4 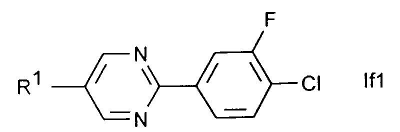 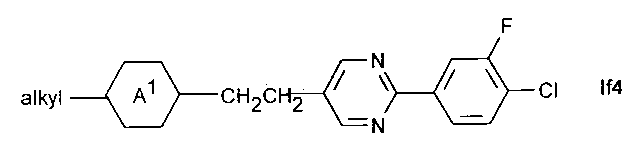 in which alkyl denotes straight-chained alkyl with 2 to 7 C atoms.

4. Liquid crystalline medium according to claim 1 characterized in that the heterocyclic compound is a compond of formula Ig 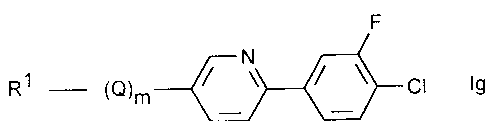 in which R1 and m have the meaning given. and
Q is 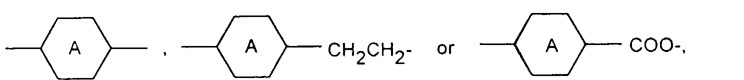 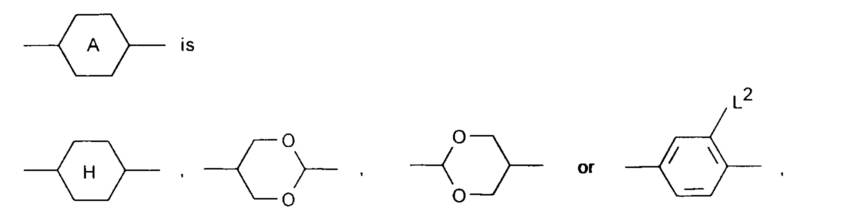
L² is H or F

5. Liquid crystalline medium according to claim 4 characterized in that the heterocyclic compound is selected from formulae Ig1 and lg3

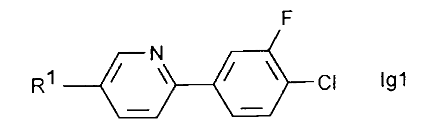

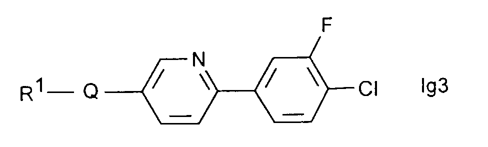

6. Electrooptical display device characterized in that it contains a liquid crystalline medium according to any of the claims 1 to 5.

## Patentansprüche

1. Flüssigkristallines Medium, bei dem es sich um eine Mischung aus mindestens zwei Verbindungen handelt, von denen mindestens eine Verbindung eine durch Chlorphenyl substitutierte Verbindung der Formel I ist, 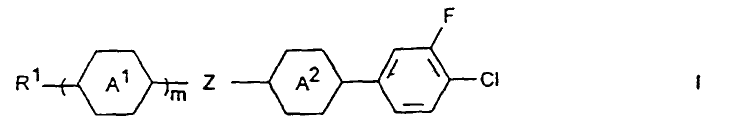 worin
R¹ einen Alkyl- oder Alkenylrest mit jeweils bis zu 16 C-Atomen, worin eine oder zwei nichtbenachbarte CH₂-Gruppen durch -O-, -S-, -CO-O- oder -O-CO- ersetzt sein können,
A² einen heterocyclischen Ring der Formel 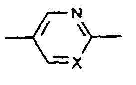 mit X gleich N oder CH, 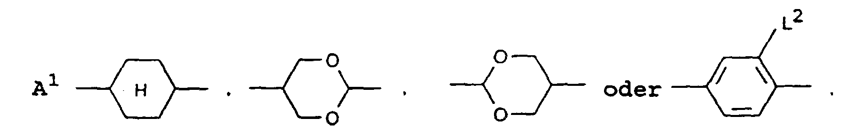
L² H oder F,
Z (CH₂)ₙ oder -COO-,
m 0 oder 1 und
n 0 oder 2 bedeuten.

2. Flüssigkristallines Medium nach Anspruch 1, dadurch gekennzeichnet, daß die heterocyclische Verbindung aus den Formeln Ia bis Ib ausgewählt ist, 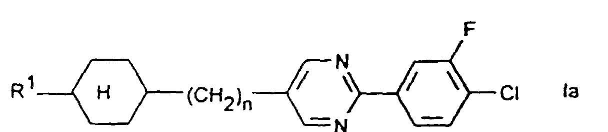 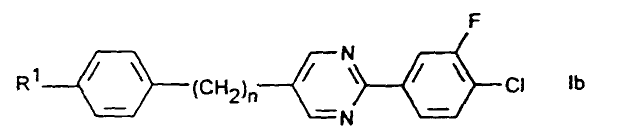 worin R¹ und n die angegebene Bedeutung besitzen.

3. Flüssigkristallines Medium nach Anspruch 1, dadurch gekennzeichnet, daß die heterocyclische Verbindung aus den Formeln If1 und If4 ausgewählt ist, 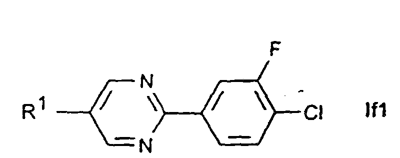 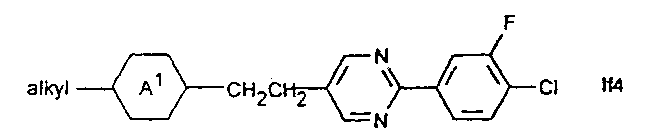 worin Alkyl geradkettiges Alkyl mit 2 bis 7 C-Atomen bedeutet.

4. Flüssigkristallines Medium nach Anspruch 1, dadurch gekennzeichnet, daß die heterocyclische Verbindung eine Verbindung der Formel Ig ist, 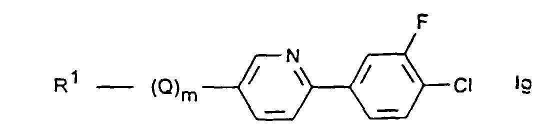 worin R¹ und m die angegebene Bedeutung besitzen und
Q 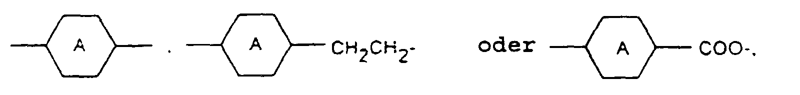 bedeutet,
wobei 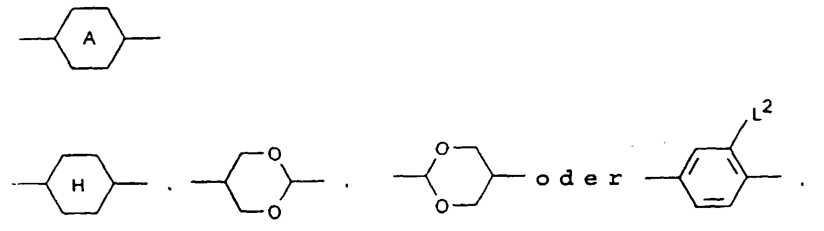 bedeutet, und
L² H oder F bedeutet.

5. Flüssigkristallines Medium nach Anspruch 4, dadurch gekennzeichnet, daß die heterocyclische Verbindung aus den Formeln Ig1 und Ig3 ausgewählt ist

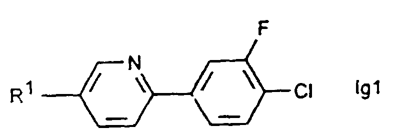

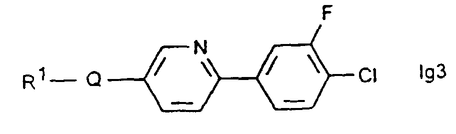

6. Elektrooptische Anzeigevorrichtung, dadurch gekennzeichnet, daß sie ein flüssigkristallines Medium nach einem der Ansprüche 1 bis 5 enthält.

## Revendications

1. Milieu de cristaux liquides qui est un mélange d'au moins deux composés où au moins un composé est un composé hétérocyclique substitué par un chlorophényle de formule I 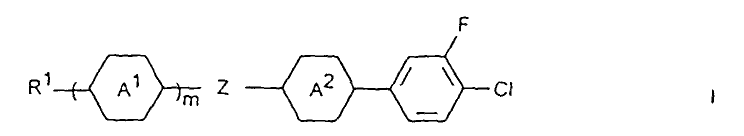 où
R¹ est un reste alkyle ou alcényle ayant jusqu'à 16 atomes de C, où un ou deux groupes CH₂ non adjacents peuvent être remplacés par -O-, -S-, -CO-O-
ou
-O-CO-,
A² est un cycle hétérocyclique de formule 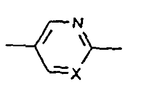 où X est N ou CH,
A¹ est 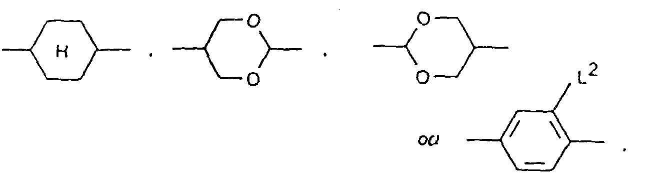
L² est H ou F,
z est (CH₂)ₙ ou -COO-,
m est 0 ou 1, et
n représente 0 ou 2.

2. Milieu de cristaux liquides selon la revendication 1, caractérisé en ce qu'on choisit le composé hétérocyclique parmi les formules Ia à Ib 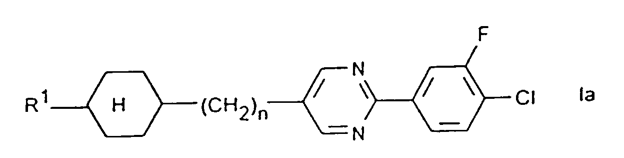 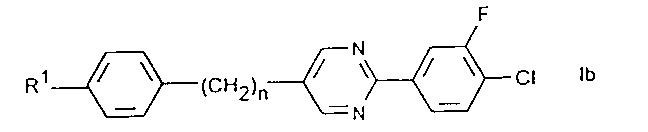 où R¹, et n ont les significations données.

3. Milieu de cristaux liquides selon la revendication 1, caractérisé en ce qu'on choisit le composé hétérocyclique parmi les formules Ifl et If4 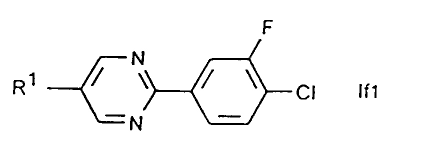 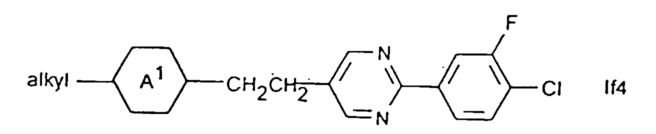 où alkyle représente un alkyle à chaîne droite avec 2 à 7 atomes de C.

4. Milieu de cristaux liquides selon la revendication 1, caractérisé en ce que le composé hétérocyclique est un composé de formule Ig 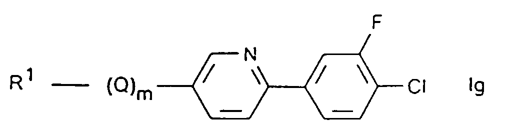 où R¹ et m ont les significations données, et
Q est 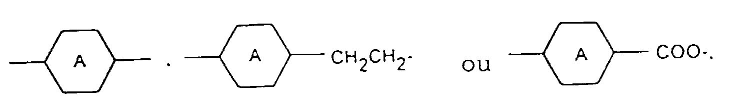 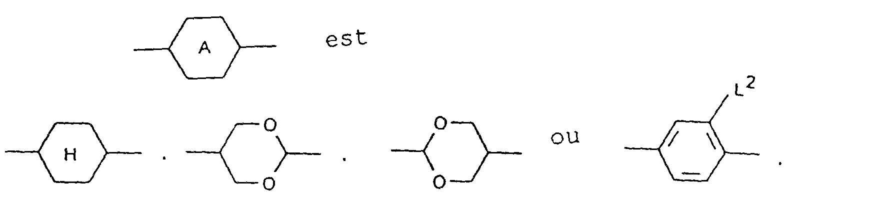
L' est H ou F.

5. Milieu de cristaux liquides selon la revendication 4, caractérisé en ce que le composé hétérocyclique est choisi parmi les formules Ig1 et Ig3

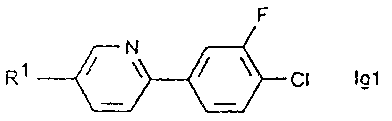

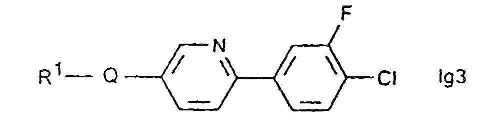

6. Dispositif d'afficheur électrooptique, caractérisé en ce qu'il contient un milieu de cristal liquide selon l'une quelconque des revendications 1 à 5.
